(19) Europäisches Patentamt
European Patent Office
Office européen des brevets

(11) **EP 3 473 264 B1**

(12) **EUROPEAN PATENT SPECIFICATION**

(45) Date of publication and mention
of the grant of the patent:
**12.05.2021 Bulletin 2021/19**

(21) Application number: **17740275.7**

(22) Date of filing: **08.06.2017**

(51) Int Cl.:
*A61K 38/18* (2006.01)          *G01N 33/50* (2006.01)
*A61P 25/28* (2006.01)

(86) International application number:
**PCT/CU2017/050005**

(87) International publication number:
**WO 2017/220053 (28.12.2017 Gazette 2017/52)**

(54) **USE OF THE BASIC FORM OF RECOMBINANT HUMAN ERYTHROPOIETIN IN THE TREATMENT OF PATIENTS WITH SPINOCEREBELLAR ATAXIA WITH CAG REPEAT MUTATIONS**

VERWENDUNG DER GRUNDFORM VON REKOMBINANTEM HUMANEM ERYTHROPOIETIN BEI DER BEHANDLUNG VON PATIENTEN MIT SPINOZEREBELLÄRER ATAXIE MIT CAG-REPEAT-MUTATIONEN

UTILISATION DE LA FORME BASIQUE DE L'ÉRYTHROPOÏÉTINE HUMAINE RECOMBINANTE DANS LE TRAITEMENT DE PATIENTS SOUFFRANT D'ATAXIE SPINOCÉRÉBELLEUSE À MUTATIONS DU TYPE RÉPÉTITIONS CAG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.06.2016 CU 20160092**

(43) Date of publication of application:
**24.04.2019 Bulletin 2019/17**

(73) Proprietor: **Centro de Inmunologia Molecular La Habana 11600 (CU)**

(72) Inventors:
• **AMARO GONZÁLEZ, Daniel Enrique**
  **La Habana 10400 (CU)**
• **VELÁZQUEZ PÉREZ, Luis Clodoaldo**
  **Holguín 80100 (CU)**
• **GARCÍA RODRÍGUEZ, Julio César**
  **Boyeros, La Habana 17200 (CU)**
• **RODRÍGUEZ LABRADA, Roberto**
  **Holguín 80100 (CU)**
• **SOSA TESTÉ, Iliana María**
  **La Habana 10400 (CU)**
• **ORTEGA SÁNCHEZ, Ricardo**
  **Holguín 80100 (CU)**
• **HERNÁNDEZ CASAÑA, Patricia**
  **La Habana 10400 (CU)**
• **GONZÁLEZ TRIANA, Consuelo**
  **Boyeros, La Habana 17200 (CU)**
• **JAY PÉREZ, Daniel**
  **La Habana 10200 (CU)**
• **LÓPEZ MATILLA, Lien**
  **La Habana 10700 (CU)**
• **JIMÉNEZ RODRÍGUEZ, Daise**
  **La Habana 10700 (CU)**

(74) Representative: **V.O.**
  **P.O. Box 87930**
  **Carnegieplein 5**
  **2508 DH Den Haag (NL)**

(56) References cited:
  **EP-A1- 1 997 483**

• **Anonymous: "NeuroEPO - Type 2 Spinocerebellar Ataxia Phase I-II | Registro Público Cubano de Ensayos Clínicos", , 29 April 2016 (2016-04-29), XP055418021, Retrieved from the Internet: URL:https://web.archive.org/web/2016042920 3917/http://registroclinico.sld.cu/en/tria ls/RPCEC00000187-En [retrieved on 2017-10-23]**

- Redaction Ahora: "Neuro EPO Clinical Trial with Ataxic Patients Assessed as Positive - Periódico AHORA", , 9 February 2017 (2017-02-09), XP055418010, Retrieved from the Internet: URL:https://web.archive.org/web/2017020903 3348/http://www.ahora.cu/en/sections/healt h/21704-neuro-epo-clinical-trial-with-atax ic-patients-assessed-as-positive [retrieved on 2017-10-23]
- See: "Use of Cuban Recombinant Human Erythropoietin in Parkinson's Disease Treatment", , 1 January 2012 (2012-01-01), XP055418007, DOI: 10.1590/S1555-79602012000100003 Retrieved from the Internet: URL:https://www.researchgate.net/profile/L azaro_Alvarez/publication/221832158_Use_of _Cuban_Recombinant_Human_Erythropoietin_i n _Parkinson's_Disease_Treatment/links/00463 52fa7ae140c82000000/Use-of-Cuban-Recombin a nt-Human-Erythropoietin-in-Parkinsons-Dise ase-Treatment.pdf?origin=publication_detai l [retrieved on 2017-10-23]
- KARL EGGER ET AL: "White Matter Changes in Patients with Friedreich Ataxia after Treatment with Erythropoietin : White matter changes after treatment with erythropoietin", JOURNAL OF NEUROIMAGING, vol. 24, no. 5, 9 September 2013 (2013-09-09), pages 504-508, XP055417895, US ISSN: 1051-2284, DOI: 10.1111/jon.12050
- LUIS VELÃ ZQUEZ-PÃ CR REZ ET AL: "A Comprehensive Review of Spinocerebellar Ataxia Type 2 in Cuba", THE CEREBELLUM, SPRINGER-VERLAG, NEW YORK, vol. 10, no. 2, 12 March 2011 (2011-03-12) , pages 184-198, XP019915509, ISSN: 1473-4230, DOI: 10.1007/S12311-011-0265-2
- MAURICE TANGUI ET AL: "Intranasal formulation of erythropoietin (EPO) showed potent protective activity against amyloid toxicity in the A[beta]25-35 non-transgenic mouse model of Alzheimer's disease.", JOURNAL OF PSYCHOPHARMACOLOGY (OXFORD, ENGLAND) NOV 2013, vol. 27, no. 11, November 2013 (2013-11), pages 1044-1057, XP009500979, ISSN: 1461-7285

**Description**

**SCOPE OF THE TECHNIQUE**

The present invention relates to the fields of Biotechnology and Medicine, particularly to the use for the treatment of Spinocerebellar Ataxia with mutations of CAG repeats type, of pharmaceutical compositions having as active ingredient the basic form of recombinant human erythropoietin and administered nasally.

**BACKGROUND**

Hereditary cerebellar ataxias constitute a health problem worldwide, which has been demonstrated in the current "genomics era" by means of the molecular findings that have been made related to these conditions. The term is used to refer to a group of diseases, most of them progressively neurodegenerative that are inherited in Mendelian character, characterized by central motor and limbic incoordination as well as oculomotor, biochemical and neurocognitive associated alterations (Koeppen A. The Cerebellum. 4: 62-73, 2005; Matilla A et al., Brain. 129: 1357-1370, 2006).

Hereditary cerebellar ataxias are divided into two large groups, Autosomal Recessive Cerebellar ataxias (ARCA) and Autosomal Dominant Cerebellar Ataxia (ADCA). The latter are characterized by their heterogeneity and include spinocerebellar ataxias (SCA). Currently 40 molecular forms of SCA are known, although the genetic heterogeneity observed among these suggests that at least 30% of its molecular etiology has not yet been identified (Schols L et al. The Lancet. 3: 291-30, 2004; Storey y otros. Semin Neurol. 34(3): 280-92, 2014). So far we have identified and characterized the mutations associated with 13 subtypes of SCA (Smeets and Verbeek, Neurobiol Dis. 88: 96-106 2016). Mutations that are most commonly related with ADCA are CAG trinucleotide expansions in the translatable region of the corresponding gene, and the ones which are presented in SCA1-3, SCA6-7, SCA-17 subtypes and DRPLA. They produce a polyglutamine expansion in the specific protein, which confers to it an anomalous dimensional structure. Under these conditions, proteins are aggregated in the form of nuclear and/or cytoplasmic inclusions and perform toxic functions in the cells where they are expressed, causing their death and consequently the neurodegenerative process associated with these diseases (Durr. The Lancet Neurology 9 (9): 885-894, 2010).

From the neuropathological point of view numerous mechanisms by which the mutated proteins induce neuronal death have been described. They comprise the interference with various molecular pathways that include the aggregation and clearance of the protein, the transcriptional regulation, the ubiquitin/proteasome system, alterations in calcium homeostasis and activation of pro-apoptotic routes. (Durr The Lancet Neurology 9 (9). 885-894, 2010).

Sympathetic hyperactivity was detected in studies of the SCA, which increases oxidative stress and reduces the neuroprotective mechanisms thus intensifying neurodegenerative phenomena, all modifiable and worsened by the number of CAG repeats and the progression of the disease (Pradhan C et al. Acta Neurol Scand. 117(5): 337-342, 2008).

Currently there is no specific treatment for any type of hereditary ataxia but symptomatic treatments are applied depending on the evolution of the disease in each patient. So far, the therapeutic alternatives used are comprehensive neuro-rehabilitation and some symptomatic palliative treatments. These alternatives have been very successful in treating the main phenotypic characteristics, however no therapies aimed at restoring the neuroprotective capabilities have been applied (Braga Neto and other Arq Neuro-Psiquiatr 74 (3): 244-252, 2016). Recent researches has shown that most of the clinical manifestations of these diseases begin several years before the cerebellar syndrome, allowing to describe a prodromal stage of these conditions, which justifies the initiation of treatment from these early stages (Velázquez-Pérez y otros. The Cerebellum 13(5): 568-79, 2014; Velázquez-Pérez et al. The Lancet Neurology. 13(5): 482-489, 2014).

The inventors of the present application discovered that in type 2 SCA patients (SCA2), the dysfunction of the endogenous neuroprotective mechanisms are due to decreased levels of erythropoietin (EPO) in the cerebrospinal fluid (CSF). These biochemical changes were more evident in patients with greater polyglutamine expansions and/or of longer duration of the disease. Thus that the measurement of these levels was found to be a valuable piece of information to be taken into account when determining the severity of the disease and deciding how to treat these patients or even when predicting whether or not these patients will be responders to a specific therapy.

In patients with hereditary ataxia it has demonstrated the effect of EPO as a neuroprotector, specifically in Friedreich's ataxia (Mariotti C et al. J Neurochem. 126 Suppl 1: 80-87, 2013; Egger Ket al. J Neuroimaging. 24(5): 504-508, 2014). Given the above findings and that in WO 2007/009404 patent application nasal formulations having as active ingredient the basic form of recombinant human erythropoietin (rhEPO), which can be used in the treatment and/or prevention of central nervous system diseases including neurodegenerative disorders, are claimed, the inventors of this application decided to evaluate the effect of such formulations in patients with ataxia caused by triplet expansions.

Surprisingly, the inventors of the present invention discovered that the basic form of rhEPO has a therapeutic effect in patients suffering from SCA with mutations of the CAG repeats type. This invention provides for the first time a novel and effective treatment for the population of SCA patients with mutations of the CAG repeats type that had not been conducted in previous treatments.

## BRIEF DESCRIPTION OF THE INVENTION

The invention is defined in the claims.

One aspect disclosed herein relates to the use of formulations containing as active principle the basic form of rhEPO and administered intranasally for the treatment of SCA with mutations of the CAG repeats type, in a particular embodiment the SCA is of type 2. Also disclosed herein is a method of stratification of SCA patients as responders or non-responders to treatment with the basic form of rhEPO that involves the administration of said rhEPO to this type of patients based on the below-mentioned findings:

- The number of CAG repeats correlates with the severity of the disease and / or,
- the number of CAG repeats correlates inversely with the concentration of EPO in the cerebrospinal fluid (CSF).

It further comprises the use of the base form of rhEPO using the stratification method mentioned above.

A particular embodiment disclosed herein comprises that non-responders patients show no improvement in SCAFI scale after a year of treatment.

The basic form of rhEPO may be administered three times per week in a dose range from 0.1 mg / ml to 4mg / ml. Particularly the basic form of rhEPO is administered for a period of 6 to 12 months and this dose regimen can be individually optimized so that:

- the number of CAG repeats correlates with disease severity and / or,
- the number of CAG repeats correlates inversely with the concentration of EPO in the CSF.

## DETAILED DESCRIPTION OF THE INVENTION

In one aspect, the present invention relates to the use of human EPO in SCA patients with mutations of the CAG repeats type. In one embodiment of the invention human EPO is recombinant and preferably the basic form of rhEPO. A blood sample from a patient to whom a series of tests was performed previously and who was clinically diagnose with SCA is obtained and the number of CAG repeats is determined.

Additionally, the present invention relates to a method of stratifying subjects who are responders to treatment with the basic form of rhEPO. This method may further comprise a step of administering an effective amount of the basic form of rhEPO to a subject. Said subject is a human patient.

Additionally, in the present invention after the subject completes the treatment with the basic form of rhEPO the concentration of EPO in the CSF is quantified again and the neurocognitive parameters related to attention, memory and fronto-executive functions are evaluated again as well as motor functions and saccadic speed.

The present invention comprises that the examples of SCA treated herein include, but are not limited to SCA1-3, SCA6-7, SCA-17 and DRPLA. Improving in the context of the present invention refers to the increase that treatment with the basic form of rhEPO administered nasally produces in EPO levels in the CSF and/or the improvement any of the neurocognitive parameters related to attention, memory and the fronto-executive functions or motor symptoms.

In the context of the present invention, the term "therapeutic effect" generally refers to a desirable or beneficial effect of treatment, for example, improvement or remission of the disease manifestations. The term "manifestation" of a disease is used herein to describe its perceptible expression, and includes both the clinical manifestations, hereinafter defined as signs of the disease that can be detected during a physical examination and / or are perceptible by the patient (i.e., symptoms), and the pathological manifestations, that is disease manifestations in the cellular and molecular level.

## Methods of clinical diagnosis of SCA.

*Neuropsychological assessment*

A battery of neuropsychological tests described in the literature is applied to study the fronto-executive functions including but not limited to:

- The test of phonological verbal fluency: the patient is ordered to find words in his vocabulary beginning with F, A and S, in a span of one minute.
- Stroop test: The test consists of three parts. In the first part, the subject must read aloud and as quickly as possible a list of names of colors (red, green and blue) printed in black ink in a sheet of paper. In the second part he is shown a second sheet and he must name the color (red, green or blue) in which a series of stimuli without linguistic value (crosses) are printed. The colors are not presented in the same order they had in the words previously shown in the first part of the test. In the third part the words of the first printed sheet are mixed with the colors of the second one (the first item of the first sheet is printed using the ink of the first item of the second sheet), so that in no case the color of the ink matches the meaning of the word. The subject must name the color of the ink, disregarding the printed word.

To assess memory:

- Spontaneous verbal memory test: Ten words are said to the subjects and then they are asked to repeat them in any order. The have up to 10 chances to recall them correctly. The number of words recalled in each trial as well as the number of trials required to recall all the words are assessed.

and to evaluate the visual motor skills

- Test of Hooper: Eight sheets with different figures divided into disorganized pieces are shown to the patient and he is asked to order them visually, as if putting together a puzzle, identifying the figure represented in each case.

*Clinical Assessment*

The international scales available to assess motor impairment of ataxia are applied. They include but are not limited to SARA scale (Scale for Assessment and Ataxia Rating) and SCAFI index (Spinocerebellar ataxia Functional Index). In SARA scale the total score and score of each item are analyzed, following the below mentioned methodology:

I. Gait:

- The patient is asked to walk at a given distance parallel to a wall including a half-turn (Turn around in the opposite direction of the gait).
- To walk in tandem without support.

    0 - Normal. No difficulties in walking, turning and walking tandem (up to one misstep allowed).
    1. Slight difficulties: only visible when walking 10 consecutive steps in tandem.
    2. Clearly abnormal, tandem walking for more than 10 steps is not possible.
    3. Considerable staggering, difficulties in half-turn, but without support.
    4. Marked staggering, intermittent support of the wall required.
    5. Severe staggering, permanent support of one stick or light support by one arm required.
    6. Walking more than 10 meters only possible with strong support (two special sticks or stroller or accompanying person).
    7. Walks less than 10 meters only with strong support (two special sticks or stroller or accompanying person).
    8. Unable to walk, even supported.

II. Stance:

- The patient is asked to stand in a natural position, with feet together in parallel and in tandem, no space between both feet. The patient should not wear shoes and eyes must remain open. For each position, up to three trials are allowed. Best trial is rated.

    0- Normal: Able to stand in tandem for more than 10 seconds.
    1- Able to stand with feet together without sway, but not in tandem for more than 10 seconds.
    2- Able to stand with feet together for more than 10 seconds, but only with sway.
    3- Able to stand for more than 10 seconds without support in natural position, but not with feet together.
    4- Able to stand for more than 10 seconds in a natural position but only with intermittent support.
    5- Able to stand more than 10 seconds in a natural position only with constant support of one arm.
    6- Unable to stand for more than 10 seconds even with constant support of one arm.

III. Sitting postural stability:

- The patient is asked to sit on an examination bed without support of feet, eyes open and arms outstretched to the front.

    0. Normal: No difficulties to sit for more than 10 seconds.

1. Slight difficulties, intermittent sway.
2. Constant sway, but able to sit for more than 10 seconds without support.
3. Able to sit for more than 10 seconds with intermittent support.
4. Unable to sit for more than 10 seconds without continuous support.

IV. Speech disturbance:

- Speech is assessed during normal conversation.

    0. Normal.
    1. Slight signs of speech disturbance
    2. Impaired speech, but easy to understand
    3. Occasional words difficult to understand
    4. Many words difficult to understand
    5. Only five words are understandable
    6. Unintelligible speech

V. Finger chase:
The patient sits comfortably, if necessary, support of feet and trunk is allowed. Examiner sits in front of the patient and performs five consecutive, sudden and fast pointing movements in unpredictable directions in a frontal plane, at about 50 % of the patient's reach. Movements have amplitude of 30 cm and a frequency of one movement every two seconds. The patient is asked to follow the movements with his index finger, as fast and precisely as possible. Each side (right and left) is evaluated. The average performance of the last 3 movements is rated and an average score of both is obtained. (right + left / 2).

    0 No dysmetria
    1 Dysmetria, hypo/hypershooting target <5 cm
    2 Dysmetria, under/ overshooting target < 15 cm
    3 Dysmetria, under/ overshooting target > 15 cm
    4 Unable to perform 5 pointing movements.

VI. Nose-finger test

- The patient sits comfortably. If necessary, support of feet and trunk is allowed. He is asked to point repeatedly with his index finger from his nose to examiner's finger which is in front of the patient at about 90 % of patient's reach. Movements are performed at moderate speed. The patient makes 5 pointing movements with each hand. Average performance of movements is rated according to the amplitude of the kinetic tremor, examiner's finger remains fixed. Both sides are measured separately and an average is calculated.

    0 No tremor
    1 Tremor with an amplitude of less than 2 cm
    2 Tremor with an amplitude of less than 5 cm
    3 Tremor with an amplitude of more than 5 cm
    4 Unable to perform 5 pointing movements

VII. Alternating hand movements.

- The patient sits comfortably. If necessary, support of feet and trunk is allowed. The patient is asked to perform 10 repetitive cycles of prone supination movements of the hands on his thighs as fast and as precise as possible. Movement is demonstrated by the examiner at a speed of approximately 10 cycles within 7 seconds. Exact times for movement execution have to be measured.

    0. Normal, no irregularities, (performs <10s).
    1. Slightly irregular (performs <10s).
    2. Clearly irregular: simple movements are difficult to distinguish or have significant interruptions, running time less than 10 seconds.
    3. Very irregular: simple movements are difficult to distinguish or have significant interruptions, running time more than 10 seconds.

4. Unable to complete the 10 cycles.

VIII. Test Heel - Knee.

The separate evaluation of each side is performed and an average of both sides is obtained. The patient lies on a bed without sight of his legs and is instructed to lift one leg, point with the heel to the opposite knee, slide down along the shin to the ankle, and lay the leg back on the examination bed. The task is performed 3 times. Slide-down movements should be performed within 1 second, if the patient slides down without contact to shin in all three trials, rate 4.

0 Normal
1 Slightly abnormal: contact to shin maintained
2 Clearly abnormal: goes off shin up to 3 times during 3 cycles
3 Severely abnormal: goes off shin 4 or more times during 3 cycles
4 Unable to perform the task.

The SCAFI has three measurements: PATA, 9HPT and 8 MW. The average of the three Z measurements is calculated.

I. 8 METERS WALKING TEST (8 MW+)

The patient is directed to one end of a 8 meters line and asked to walk the 8 meters distance to the other end as quickly as possible but without actually running. Exact time is recorded with a stopwatch. The value in seconds is recorded with a number after the decimal point (eg 5.2 seconds). Then the patient is asked to perform the same task, and the time is taken again using the stopwatch. Exact time is recorded. At the end of the trial the mean of both times is obtained first and then the reciprocal of this average is calculated. It will also be recorded if an assistive device for walking was needed and if it was necessary to repeat some trial.

II. Timed dexterity test: 9-hole peg test (9HPT-0)

The patient is asked to place to pick up the 9 pegs, using one hand only, one at a time, and put them into the 9 holes as quickly as he can in any order that he wishes until all the holes are filled. He should execute the task first with his dominant hand and then with the non-dominant hand. The task is done twice with each hand and the average for each hand is calculated and then the reciprocal of these averages. With each reciprocal separately the average of these reciprocal is calculated and this value is used for the scale.

III. Timed speech task: PATA rate

The patient is asked to repeat "PATA" as quickly and distinctly as possible for 10 seconds until told to stop. The test is performed two times without major pause (no more than 5 min) in between. The measurements of the patient in each case are recorded with a computer and the number of repetitions of the word afterwards carefully counted. The average of these repeats is calculated and this value is the one used on the scale.

The calculation of each Z score is performed as follows:

$$8 \text{ MW-Z} = (1/8 \text{ MW} - \text{Average } 1/8 \text{ MW basal}) / \text{Standard Deviation } 1/8 \text{ MW baseline.}$$

$$9\text{HPT-Z} = (1 / 9\text{HPT} - \text{Average } 1/9 \text{ HPT basal}) / \text{Standard Deviation } 1/9 \text{ HPT baseline.}$$

$$\text{PATA-Z} = (\text{Prom PATA} - \text{Prom PATA baseline}) / \text{Average Standard Deviation PATA baseline.}$$

These measurements of runtimes for the three Z tests are performed using a digital stopwatch, properly calibrated.

*Electronystagmography assessment of saccadic eye movements.*

The movement in the horizontal plane is recorded binocularly with chlorinated silver electrodes, placed on the outer edge of both eyes. To register vertical movements, an electrode was placed over the right superior orbital rim and the other below it. A filtering band was used between 0.02 to 70 Hz, a sensitivity of $200\mu V/$ division, a time of analysis of 1000 milliseconds, a time constant of eight seconds and a sampling frequency of 200 Hz.

Saccadic eye movements were evoked by using a white circular stimulus on a black background. The patient was asked to move his eyes and follow as quickly as possible the stimulus, without moving his head. To avoid these movements and control the distance between the patient and the monitor, the head and chin are fixed with a suitable supporting device. Ten saccadic horizontal centrifuges for stimuli at 10°, 30° and 60° of amplitude were recorded. Calibrations were performed at 30 ° before and after each recording. To quantify the saccadic speed an automatic analysis of electronystagmographic signals was performed, followed by manual correction of the position of the cursors of start and end of each saccade was performed.

## Methods of molecular identification of the SCA

*Determining the amount of CAG repeats.*

This determination is made by means of the polymerase chain reaction (PCR). Sources of sample collection for such determination include but are not limited to peripheral blood.

*Determination of EPO concentration in the CSF.*

The determination of EPO in the CSF can be performed using various techniques which include but are not limited to ELISA, HPLC, Lowry.

## Pharmaceutical compositions

The neuroprotective pharmaceutical formulations mentioned in the present invention are administered nasally and are in the form of aqueous solutions which finished form are nasal drops or nasal spray. These pharmaceutical formulations comprise the basic form of rhEPO as active ingredient and optionally excipients and/or pharmaceutically suitable stabilizers. In another variant of the present invention formulations including all the above components except the active ingredient can be found. These formulations will be hereinafter referred to as placebo.

The basic form of rhEPO refers to any rhEPO molecule that contains in its structure less than nine sialic acid residues per protein molecule.

The excipients and/or suitable stabilizers are nontoxic to the recipients at the dosages and concentrations employed and include bioadhesive polymers such as hydroxymethylcellulose, hydroxypropylcellulose and methylcellulose; protein stabilizers such as L-tryptophan, L-leucine, L-arginine hydrochloride and/or L-histidine hydrochloride and its salts.

## Forms of administration of the basic form of rhEpo.

The invention includes a method that comprises the administration to the patient of the basic form of rhEPO from one to three times per week every for periods of time from 6 to 12 months. The administration will be performed intranasally, slowly, by instilling the drug in the mucosa. The dose range to be administered is from 0.1 mg/ml to 4mg/ml, preferably 0.5mg/ml-2mg/ml. The maximum volume to be administered each day of treatment is 1 ml, 0.5 ml by each nostril. This volume can be administered in smaller volumes with time intervals of 10-20 minutes between each application, preferably every 15 minutes.

## BRIEF DESCRIPTION OF THE FIGURES

**Figure 1.** Qualitative analysis of the images of the Clasping Test showing the position taken by the animals of each group. As can be seen the position differed according to the estimate of the area between the four limbs and the distance from the axis of each animal.

**Figure 2.** Comparison of survival of the different study groups. Statistically significant differences between the group of ataxic animals treated with placebo and the group of animals treated with the nasal formulation of the basic form of the hrEPO and healthy controls are observed, these differences become more evident as time goes by, no differences between the group of ataxic mice treated with hrEPO and the healthy control were observed. Different letters were used for statistically significant differences.

**Figure 3a.** Decreased levels of EPO in the CSF of patients with SCA2, significant differences between SCA2 patients and healthy controls are shown.

**Figure 3b.** Relationship between decreased EPO levels in the CSF with molecular damage. An inverse correlation is observed, that is, as the number of CAG repeats in SCA2 patients increases, EPO levels decrease in the CSF.

**Figure 4.** EPO concentration measured by ELISA in the CSF of the patients analyzed.

## EXAMPLES

### Example 1. Demonstration of the neuroprotective effect of rhEPO in the nasal formulation of SCA type-2 ataxic mice.

Twenty hybrid mice weaned at 21 days of age, descendants of parental males of the OF1 line and females of B6D2F1 line, homozygous dominant for SCA-2 gene were used. They were separated by sex into 2 groups, 10 females and 10 males, and these two groups further divided into two experimental groups. As healthy controls of the experiment five male mice of the OF1 line and five female mice of line B6D2F1 were used.

A 1mg/ml of the basic form of rhEPO was administered nasally to one of the groups of ataxic female mice and one of the groups of ataxic males. The remaining two groups of ataxic animals were administered placebo. This administration was performed on alternate days, during morning time for 12 months. Healthy controls received no treatment.

The neurological behavior of the animals and clinical course of the disease was analyzed. The neurological behavior was studied by means of the Clasping Test, in order to perform this test the animals received training for a time period of 30 days prior to it. The protocol of the test consists of hanging the animal by the tail with his head down and analyzing the position adopted, taking into account the position of the limbs with relation to the axis (position of equilibrium or fetal), and a qualitative assessment by images. The test was performed every month to analyze the equilibrium position. The clinical course of the disease was analyzed by using the parameter survival.

For statistical processing of the results the Statgraphics Plus 5.1 software was used.

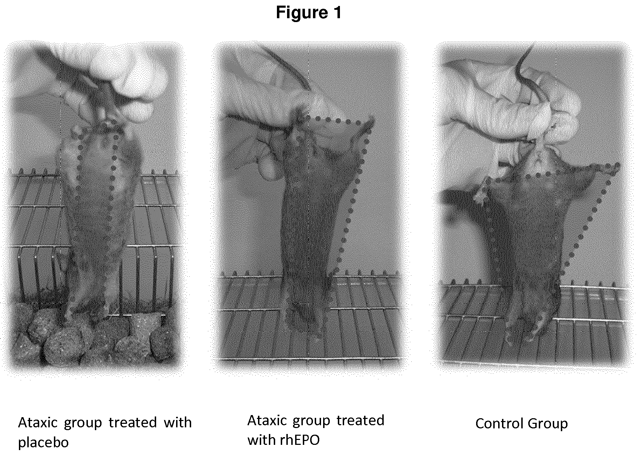

Figure 1 shows images of the position taken by the animals in each group. Ataxic animals treated with placebo mostly adopted a position similar to fetal position showing reduced mobility; healthy controls adopted various unstable positions with spread legs, constantly seeking equilibrium by moving the legs; and the animals treated with the nasal formulation of rhEPO, showed higher variability of positions, going from fetal position to spread legs position, with a majority of mice adopting intermediate positions.

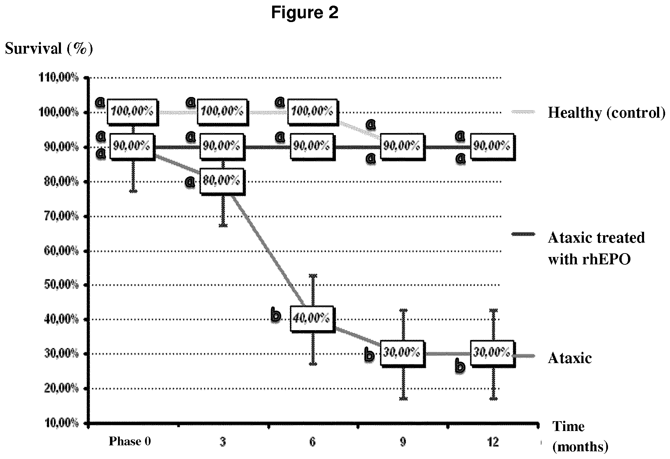

Figure 2 shows survival in the different animal groups. As shown by the figure as times goes by the differences between the group of untreated ataxic animals and the other two groups significantly increase. The result obtained is obviously attributable to the described evolution of the disease and confirms the effectiveness of the treatment with rhEPO in modifying the clinical course of the disease.

### Example 2. Quantification of the number of CAG repeats in patients with SCA2.

DNA was isolated from 10 ml of peripheral blood of each patient. Amplification of the repetitive sequence of CAG in SCA2 locus was performed by PCR using for this purpose the DAN1 (5'-cgtgcgagccggtgtatggg-3', C and 5 fluorescence) and UH10 (5'-ggcgacgctagaaggccgct-3') primers.

For the determination of the exact number of CAG repeats an analysis of fragments was performed, for this purpose 4 $\mu$l of products from the PCR were combined with 3 $\mu$l of loading dye and 1 $\mu$l of each ALFexpress Sizer of 100 and 300 base pairs (Amersham Biosciences).

This mixture was heated at 95°C for three minutes before separation with 8% ReproGel gel electrophoresis using a gene sequencer (ALFexpress II, Amersham Biosciences). Size of CAG repeats was determined by comparison using an ALFexpress Sizer of 50-500 base pairs, which has a collection of ten kinds of fragments differentiated by increments of 10 base pairs as an external standard of molecular weight and an ALFexpress Sizer of 100 and 300 base pairs as internal standard of molecular weight. To control the electrophoretic run and data collection ALFwin™ Sequence Analyser 2.00 software was used and for data analysis and estimation of the allele size AlleleLinks™ 1.00 (Amersham Biosciences) software.

An average of 39.4 CAG repeats was obtained, the minimum number of repetitions was 35 and the maximum was 55.

**Example 3. Decreased levels of EPO in the CSF of patients with SCA2.**

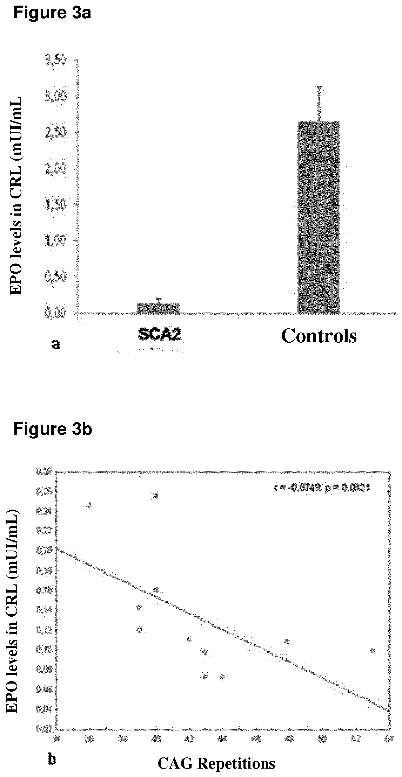

EPO concentration in CSF was determined in a sample of 20 patients with SCA2 and in 20 healthy controls by *Sandwich* ELISA (EPO ELISA version 08, Roche). The results showed a significant decrease (mean: 0.12 mIU/mL), which represents 95% decrease of normal levels of EPO in the CSF as compared to the control group (mean: 2.65 mIU/mL) (Figure 3a). Forty-five percent of the patients analyzed had values below the detection limit of the technique and in the rest the values had decreased as compared to healthy controls, the highest concentration value obtained in the group of patients with SCA2 was 0,396 mIU /mL. These biochemical changes are more evident in patients with greater polyglutamine expansions (Figure 3b) and/or longer duration of disease.

**Example 4. Evaluation of the effect of treatment with the nasal formulation of the basic form of rhEPO in patients with SCA2.**

A phase I-IIa, single-center, randomized, placebo-controlled, double-blind clinical trial, in patients with a clinical diagnosis of SCA2 and molecularly with a quantity higher or equal to 32 repetitions of the trinucleotide CAG, in early stages (I-II) of the disease was performed. In the trial 34 patients were randomly divided into two study groups; a group 17 patients received the basic form of rhEPO and the other 17 patients received placebo.

RhEPO was administered nasally to a concentration of 1mg/mL three times a week, until six months of treatment were completed. A dose of 0.25ml was applied first in each nostril and 15 minutes later a second dose of the same volume was again applied, for a final volume in each nostril of 0.5 mL and a total volume of 1 mL administered each day of treatment.

The primary endpoint to measure the effect of the product administered was the SCA Functional Index (SCAFI). Patients were evaluated before the beginning and end of the treatment stage and changes were determined by means of the SCAFI. The decrease in time of the average of the three scores (Z) that comprise it allows to monitor the progression of the disease.

The data analysis shows that at the end of treatment, in the group of patients treated with the basic form of rhEPO there is an increase in the values of the mean of the differences of the SCAFI, due to the decrease of values of this scale at end of treatment; contrary to what happens in the placebo group. The differences between the means of both groups are statistically significant (Table 1). The number of patients who completed the trial was different from baseline due to voluntary abandonment of the trial and in two cases due to treatment discontinuation caused by the progression of the disease, in both cases the patients who discontinued treatment coincided with the ones who had 52 and 55 CAG triplet repeats.

Table 1. Results of measurement of SCAFI parameter before and after completion of the trial.

| Variable | Group | n | Mean of differences | Significance |
|----------|-------|---|---------------------|--------------|
| SCAFI | Treated with rhEPO | 13 | 0,75 | 0,04 |
| | Placebo | 16 | 0,41 | |

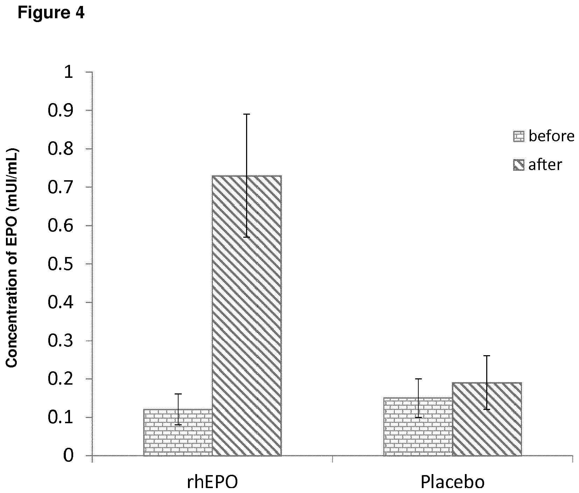

EPO concentration in the CSF of the patients was determined by *Sandwich* ELISA (EPO ELISA version 08, Roche). Figure 4 shows the increase of EPO concentration in patients treated with the basic form of rhEPO as compared to the levels they had before treatment and to those of patients who received the placebo.

These results indicate that in the group treated with the basic form of the rhEPO there is a significant clinical improvement associated with improvement of motor symptoms which evidences the influence of treatment on the most significant parameters that characterize the disease and consequently in the deceleration of disease progression.

**Claims**

1. The basic form of recombinant human erythropoietin (rhEPO) for use in a method of treating spinocerebellar ataxia (SCA) with mutations of the CAG repeats type; wherein the basic form of rhEPO is for intranasal administration; and wherein the SCA is type 2.

2. The basic form of rhEPO for use according to claim 1, wherein the basic form of rhEPO is for administration in a dose ranging from 0.1 mg/ml to 4 mg/ml.

3. The basic form of rhEPO for use according to claim 1 or claim 2, wherein the basic form of rhEPO is for administration from one to three times a week for a time period of 6 to 12 months.

4. A method of stratification of SCA type 2 patients into responders or non-responders to intranasal treatment with the basic form of rhEPO, which involves identifying patients as responders if the concentration of EPO in a sample of cerebrospinal fluid (CSF) obtained from said patient is decreased as compared to a healthy control.

5. The basic form of rhEPO for use according to claim 1, wherein the basic form of rhEPO is for administration to SCA type 2 patients that are identified as responders according to the method of claim 4.

**Patentansprüche**

1. Basische Form von rekombinantem humanem Erythropoietin (rhEPO) zur Verwendung in einem Verfahren zum Behandeln der spinozerebellären Ataxie (SCA) mit Mutationen des CAG-Wiederholungstypen; wobei die basische Form von rhEPO zur intranasalen Verabreichung ist; und wobei die SCA Typ 2 ist.

2. Basische Form von rhEPO zur Verwendung nach Anspruch 1, wobei die basische Form von rhEPO zur Verabreichung in einer Dosis im Bereich von 0,1 mg/ml bis 4 mg/ml ist.

3. Basische Form von rhEPO zur Verwendung nach Anspruch 1 oder Anspruch 2, wobei die basische Form von rhEPO zur Verabreichung von einbis dreimal pro Woche über einen Zeitraum von 6 bis 12 Monaten ist.

4. Verfahren zur Stratifizierung von SCA-2-Patienten in Responder oder Nicht-Responder auf intranasale Behandlung mit der basischen Form von rhEPO, was Identifizieren von Patienten als Responder beinhaltet, wenn die Konzentration von EPO in einer Probe von Zerebrospinalflüssigkeit (CSF), erhalten von dem Patienten im Vergleich zu einer gesunden Kontrolle vermindert ist.

5. Basische Form von rhEPO zur Verwendung nach Anspruch 1, wobei die basische Form von rhEPO zur Verabreichung an SCA-Typ-2-Patienten ist, die nach dem Verfahren von Anspruch 4 als Responder identifiziert werden.

**Revendications**

1. Forme basique d'érythropoïétine humaine recombinante (rhEPO) à utiliser dans un procédé de traitement de l'ataxie spinocérébelleuse (SCA) avec des mutations du type répétitions CAG ; dans laquelle la forme basique de rhEPO est destinée à une administration intranasale ; et dans laquelle la SCA est de type 2.

2. Forme basique de rhEPO à utiliser selon la revendication 1, dans laquelle la forme basique de rhEPO est destinée à être administrée à une dose dans la plage de 0,1 mg/ml à 4 mg/ml.

3. Forme basique de rhEPO à utiliser selon la revendication 1 ou la revendication 2, dans laquelle la forme basique de rhEPO est destinée à être administrée une à trois fois par semaine pendant une période de 6 à 12 mois.

4. Procédé de classement de patients atteints de SCA de type 2 en répondants ou non-répondants au traitement intranasal avec la forme basique de rhEPO, qui consiste à identifier des patients en tant que répondants si la concentration d'EPO dans un échantillon de liquide céphalo-rachidien (LCR) obtenu auprès dudit patient est diminué par rapport à un témoin sain.

5. Forme basique de rhEPO à utiliser selon la revendication 1, dans laquelle la forme basique de rhEPO est destinée à être administrée à des patients atteints de SCA de type 2 qui sont identifiés comme répondants selon le procédé de la revendication 4.

## Figure 1

Ataxic group treated with placebo

Ataxic group treated with rhEPO

Control Group

## Figure 2

**Figure 3a**

**Figure 3b**

**Figure 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007009404 A

**Non-patent literature cited in the description**

- **KOEPPEN A.** *The Cerebellum,* 2005, vol. 4, 62-73
- **MATILLA A et al.** *Brain.,* 2006, vol. 129, 1357-1370
- **SCHOLS L et al.** *The Lancet.,* 2004, vol. 3, 291-30
- **STOREY.** *Semin Neurol.,* 2014, vol. 34 (3), 280-92
- **SMEETS ; VERBEEK.** *Neurobiol Dis.,* 2016, vol. 88, 96-106
- **DURR.** *The Lancet Neurology,* 2010, vol. 9 (9), 885-894
- **PRADHAN C et al.** *Acta Neurol Scand.,* 2008, vol. 117 (5), 337-342
- **BRAGA NETO.** *Arq Neuro-Psiquiatr,* 2016, vol. 74 (3), 244-252
- **VELÁZQUEZ-PÉREZ Y OTROS.** *The Cerebellum,* 2014, vol. 13 (5), 568-79
- **VELÁZQUEZ-PÉREZ et al.** *The Lancet Neurology,* 2014, vol. 13 (5), 482-489
- **MARIOTTI C et al.** *J Neurochem.,* 2013, vol. 126 (1), 80-87
- **EGGER K et al.** *J Neuroimaging,* 2014, vol. 24 (5), 504-508